(19) **Europäisches Patentamt**
**European Patent Office**
**Office européen des brevets**

(11) **EP 2 772 751 B1**

(12) **EUROPEAN PATENT SPECIFICATION**

(45) Date of publication and mention
of the grant of the patent:
**19.05.2021   Bulletin 2021/20**

(51) Int Cl.:
*G01N 21/64* (2006.01)     *C12M 1/34* (2006.01)
*C12Q 1/68* (2018.01)     *G01N 33/53* (2006.01)
*G01N 33/543* (2006.01)

(21) Application number: **12843477.6**

(22) Date of filing: **11.10.2012**

(86) International application number:
**PCT/JP2012/006515**

(87) International publication number:
**WO 2013/061529 (02.05.2013 Gazette 2013/18)**

(54) **CHEMICAL SENSOR, BIOMOLECULE DETECTION DEVICE, AND BIOMOLECULE DETECTION METHOD**

CHEMISCHER SENSOR, VORRICHTUNG ZUR ERKENNUNG VON BIOMOLEKÜLEN UND VERFAHREN ZUR ERKENNUNG VON BIOMOLEKÜLEN

CAPTEUR CHIMIQUE, DISPOSITIF DE DÉTECTION DE BIOMOLÉCULES ET PROCÉDÉ DE DÉTECTION DE BIOMOLÉCULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2011   JP 2011233003**

(43) Date of publication of application:
**03.09.2014   Bulletin 2014/36**

(73) Proprietor: **Sony Corporation**
**Tokyo 108-0075 (JP)**

(72) Inventors:
• **OZAWA, Ken**
**Tokyo 108-0075 (JP)**
• **MATSUZAWA, Nobuyuki**
**Tokyo 108-0075 (JP)**
• **MAEDA, Kensaku**
**Tokyo 108-0075 (JP)**

• **MORIYA, Yusuke**
**Tokyo 108-0075 (JP)**

(74) Representative: **D Young & Co LLP**
**120 Holborn**
**London EC1N 2DY (GB)**

(56) References cited:
EP-A2- 2 221 606     WO-A1-01/03833
WO-A1-02/063259     WO-A1-2008/140158
WO-A1-2011/103504     JP-A- H07 508 831
JP-A- H09 512 901     JP-A- 2000 508 768
JP-A- 2002 118 245     JP-A- 2002 350 348
JP-A- 2002 350 349     JP-A- 2003 161 699
JP-A- 2003 518 924     JP-A- 2004 205 335
JP-A- 2004 205 340     JP-A- 2006 004 991
JP-A- 2006 071 417     US-A1- 2002 182 716
US-A1- 2008 081 769

**Description**

Technical Field

The present technology relates to a chemical sensor for detecting a biomolecule on the basis of fluorescence emission, a biomolecule detection apparatus on which the chemical sensor is mounted, and a biomolecule detection method that uses the biomolecule detection apparatus.

Background Art

In recent years, in the fields of medicine, biochemistry, molecular biology, and the like, it has become important to detect biomolecules such as a protein, various antigen molecules, DNA (deoxyribonucleic acid), and RNA (ribo nucleic acid). In particular, the amount of samples of those biomolecules is very small, from pmol to fmol order, depending on cases, so a development of a highly sensitive and highly accurate detection method is demanded.

As a highly sensitive detection method, a method of detecting fluorescence is most typically used. In the detection method of the fluorescence, for example, a target material to be detected is labeled in advance with a fluorescent marker, and an optical sensor to which a probe material that mutually acts with the target material specifically is fixed is used to detect the fluorescence from the target material that is adsorbed to the probe material.

For example, Patent Document 1 discloses an organic molecule detection semiconductor element in which a silicon substrate on which an organic molecule probe disposition area is formed and a solid state image pickup element are integrated with each other. The element has the structure in which fluorescence generated by binding a target material to an organic molecule probe disposed in the organic molecule probe disposition area is detected by the solid state image pickup element.

Further, Patent Document 2 discloses a biopolymer analysis chip in which a micro lens is provided between spots formed of a double gate transistor (photoelectric conversion element) and a probe material. On the chip, fluorescence generated from the target material that is bound to the probe material is collected by the micro lens and is detected by the double gate transistor.

Patent Document 1: Japanese Patent Application Laid-open No. 2002-202303

Patent Document 2: Japanese Patent Application Laid-open No. 2006-4991

WO 2011/103504 describes an analytical assembly within a unified device structure for integration into an analytical system. The analytical assembly is scalable and includes a plurality of analytical devices, each of which includes a reaction cell, an optical sensor, and at least one optical element positioned in optical communication with both the reaction cell and the sensor and which delivers optical signals from the cell to the sensor. Additional elements are optionally integrated into the analytical assembly. Methods for forming and operating the analytical system are also disclosed.

US 2008/0081769 A1 describes a biosensor array, system and method for affinity based assays that are able to simultaneously obtain high quality measurements of the binding characteristics of multiple analytes, and that are able to determine the amounts of those analytes in solution. A fully integrated bioarray for detecting real-time characteristics of affinity based assays is also disclosed.

Summary of Invention

Problem to be solved by the Invention

In the structure disclosed in Patent Document 1, however, an optical system is not provided which guides isotropic light emitted from the organic molecule probe to the solid state image pickup element, so it is impossible to obtain a sufficient light quantity. Therefore, the sensitivity is low, and the accuracy is degraded. Further, the isotropic light may enter an adjacent solid state image pickup element, and a crosstalk may be generated in a detection signal. Furthermore, a material of a surface to which the organic molecule probe is bound is not defined, and an improvement of detection accuracy by uniformly binding the organic molecule probe to the surface is not achieved.

In addition, in the structure disclosed in Patent Document 2, on an upper surface of the micro lens, a light transmissive top gate electrode is formed. Such a top gate electrode is thought to be formed of an ITO (Indium Tin Oxide), graphene, or the like as a light transmissive electrode material. However, to obtain a low resistance, those materials have to have a large film thickness. This may degrade a light transmittance of the film and cause sensitivity degradation.

In view of the above-mentioned circumstances, an object of the present technology is to provide a chemical sensor, a biomolecule detection apparatus, and a biomolecule detection method capable of detecting a biomolecule with high accuracy.

Means for solving the Problem

Particular and preferred aspects of the present invention are described in the appended claims.

To achieve the above-mentioned object, a chemical sensor according to an embodiment of the present technology includes a substrate, an optical layer, and an intermediate layer.

On the substrate, a plurality of photodiodes are arranged in a planar form.

The optical layer is laminated on the substrate, and the optical layer has a waveguide formed therein which guides fluorescence to each of the photodiodes.

The intermediate layer is laminated on the optical layer, and the intermediate layer has a probe holding area formed thereon for each waveguide. The probe holding area is capable of holding a probe material.

With this structure, the fluorescence caused by the biding of the probe material held on the probe holding area to the target material, which is specifically bound to the probe material, is detected by the photodiodes. At this time, the fluorescence is guided by the waveguides provided for each photodiode, so it is possible to detect the fluorescence with high accuracy.

The waveguide may be surrounded by a reflection surface having light reflectiveness.

With this structure, the fluorescence that enters the waveguide is reflected on the reflection surface, and the fluorescence can be detected irrespective of the angle of incidence. Further, the reflection surface can prevent the fluorescence from reaching the adjacent cells.

The waveguide may have a tapered shape in such a manner that a diameter thereof is gradually reduced from the intermediate layer side to the photodiode side.

With this structure, it is possible to condense the fluorescence to the photodiodes while guiding the fluorescence that emits light isotropically to the waveguides in a wide angle range.

The waveguide has a spectral filter formed therein which attenuates excitation light and is made of a spectral material that causes the fluorescence to pass therethrough.

With this structure, it is possible to prevent the excitation light from reaching the photodiodes. The excitation light is emitted to the chemical sensor for generating the fluorescence and has to be zero ideally in the detection by the photodiodes. With this structure, it is possible to cause only the fluorescence to reach the photodiodes by the spectral filter.

The spectral filter is a color filter that causes a part of a wavelength of the fluorescence to pass therethrough.

With this structure, it is possible to cause only the photodiodes to reach the photodiodes by the color filter.

The color filter has a transmission wavelength different from that formed in the waveguide adjacent thereto.

With this structure, it is possible to perform sensing of biomolecules with changed excitation wavelengths and thus perform a highly accurate analysis by a multidirectional analysis.

The probe holding area is formed to have such a size as to be opposed to the waveguide.

With this structure, the probe holding area, that is, the fluorescence generation area is smaller than an incidence opening of the waveguide, so it is possible to guide most of the fluorescence to the waveguide and prevent the fluorescence from reaching the adjacent cells.

The probe holding area may be formed with respect to a part of the waveguide.

With this structure, it is possible to use the cells for each of which the probe holding area is not formed as reference of excitation light leaked. As described above, the excitation light is attenuated by the spectral filter but may be completely attenuated. Therefore, by referencing a signal of the cell for which the probe holding area is not formed, that is, the fluorescence is not generated, a fluorescence detection signal can be corrected.

The chemical sensor may further include an adhesive layer made of a biomolecule adhesive formed on the probe holding area.

With this structure, it is possible for a user to cause any probe material to adhere to the adhesive layer to be used.

The chemical sensor may further include a probe material layer made of a probe material adhered onto the adhesive layer.

With this structure, a measurement sample containing the target material is brought into contact with the chemical sensor, thereby making it possible to detect the target material.

The probe material may be one of DNA, RNA, a protein, and an antigen.

With this structure, it is possible to detect the target material that is specifically bound to the probe material.

In order to achieve the object described above, a biomolecule detection apparatus according to an embodiment of the present technology includes a chemical sensor and a signal processing circuit.

The chemical sensor includes a substrate on which a plurality of photodiodes are arranged in a planar form, an optical layer laminated on the substrate, and an intermediate layer laminated on the optical layer, the optical layer having a waveguide formed therein which guides fluorescence to each of the photodiodes, the intermediate layer having a probe holding area formed thereon for each waveguide, the probe holding area being capable of holding a probe material.

The signal processing circuit processes an output signal of each of the photodiodes of the chemical sensor.

With this structure, the fluorescence caused by the biding of the probe material held on the probe holding area to the target material, which is specifically bound to the probe material, is detected by the photodiodes. At this time, the fluorescence is guided by the waveguides provided for each photodiode, so it is possible to detect the fluorescence with high accuracy.

The signal processing circuit may extract a difference between the output signals of the photodiode for which the probe holding area is provided and the photodiode for which the probe holding area is not provided, as a signal corresponding to the fluorescence.

With this structure, the signal processing circuit references a signal of a cell for which the probe holding area is not formed, that is, the fluorescence is not generated, thereby making it possible to correct a fluorescence detection signal.

The signal processing circuit may set the output signal of the photodiode for which light is shielded as a reference signal.

With this structure, it is possible to use the output signal of the photodiode for which the light is shielded for definition of a black level of the photodiode.

To achieve the object described above, a biomolecule detection method according to an embodiment of the present technology includes preparing a chemical sensor including a substrate on which a plurality of photodiodes are arranged in a planar form, an optical layer laminated on the substrate, and an intermediate layer laminated on the optical layer, the optical layer having a waveguide formed therein which guides fluorescence to each of the photodiodes, the intermediate layer having a probe holding area formed thereon for each waveguide, the probe holding area being capable of holding a probe material.

An adhesive layer made of a biomolecule adhesive is formed on the probe holding area.

Different probe materials are caused to adhere to the adhesive layer to form a probe material layer.

A measurement target substance is brought into contact with the probe material layer to cause a target material contained in the measurement target substance to bind to the probe material;

The measurement target substance that is not bound to the target material is removed.

Fluorescence caused by binding of the target material to the probe material is detected by the photodiodes.

With this structure, the fluorescence caused by the binding of the target material to the probe material is guided to the photodiodes by the waveguides, so it is possible to detect the fluorescence with high accuracy.

The waveguide may be surrounded by a reflection surface having light reflectiveness, the waveguide may have a spectral filter formed therein which attenuates excitation light and is made of a spectral material that causes the fluorescence to pass therethrough, and in the step of detecting the fluorescence, the chemical sensor may be irradiated with the excitation light with one of oblique-incidence light and annular illumination light.

With this structure, it is possible to detect the fluorescence with high accuracy. The chemical sensor includes the waveguide in which the spectral filter is formed and the reflection surface that surrounds the waveguide, so the excitation light that enters the waveguide in an oblique direction is travelled in the spectral filter by a longer distance as compared to the case of entering in a vertical direction and is thus attenuated to a larger extent. That is, by making the excitation light to be the oblique-incidence light or the annular illumination light, the attenuation of the excitation is encouraged, and an SN (signal/noise) ratio can be improved.

In the step of detecting the fluorescence, changes in wavelength and brightness of the fluorescence caused by an interaction of the target material and the probe material fluorescently labeled in advance may be detected by the chemical sensor.

With this structure, it is possible to detect the changes in the wavelength and the brightness of the fluorescence caused by the interaction of the probe material and the target material with high accuracy.

In the step of detecting the fluorescence, the fluorescence caused by the target material fluorescently labeled in advance, which is bound to the probe material, may be detected by the chemical sensor.

With this structure, it is possible to detect the fluorescence caused by the fluorescently labeled target material bound to the probe material with high accuracy.

In the step of detecting the fluorescence, fluorescence labeling is performed with respect to a bound body of the probe material and the target material, and the fluorescence may be detected by the chemical sensor.

With this structure, it is possible to detect the fluorescence caused by the bound body of the probe material and the target material with high accuracy.

Effect of the Invention

As described above, according to the present technology, it is possible to provide the chemical sensor, the biomolecule detection apparatus, and the biomolecule detection method capable of detecting a biomolecule with high accuracy.

Brief Description of Drawings

[Fig. 1] A schematic diagram showing the structure of a chemical sensor of a biomolecule detection apparatus according to a first embodiment of the present technology.

[Fig. 2] A schematic diagram showing the structure of the chemical sensor of the biomolecule detection apparatus.

[Fig. 3] A perspective view showing the structure of the chemical sensor of the biomolecule detection apparatus.

[Fig. 4] Schematic diagrams showing a method of producing the chemical sensor of the biomolecule detection apparatus.

[Fig. 5] Schematic diagrams showing a method of producing the chemical sensor of the biomolecule detection apparatus.

[Fig. 6] Schematic diagrams showing a method of producing the chemical sensor of the biomolecule detection apparatus.

[Fig. 7] Schematic diagrams showing a method of producing the chemical sensor of the biomolecule detection apparatus.

[Fig. 8] A graph showing a simulation result by the chemical sensor of the biomolecule detection apparatus.

[Fig. 9] A schematic diagram showing a cell arrangement of the chemical sensor of the biomolecule detection apparatus.

[Fig. 10] A schematic diagram showing an illumination apparatus suitable for the chemical sensor of the biomolecule detection apparatus.

[Fig. 11] Schematic diagrams each showing the shape of a diaphragm of the illumination apparatus suitable for the chemical sensor of the biomolecule detection apparatus.

[Fig. 12] A schematic diagram showing the shape of illumination light by the illumination apparatus suitable for the chemical sensor of the biomolecule detection apparatus.

[Fig. 13] A schematic diagram showing a cell arrangement of a chemical sensor of a biomolecule detection apparatus according to a second embodiment of the present technology.

[Fig. 14] A schematic diagram showing a cell arrangement of a chemical sensor of a biomolecule detection apparatus according to a third embodiment of the present technology.

Best Mode(s) for Carrying Out the Invention

(First embodiment)

A biomolecule detection apparatus according to a first embodiment of the present technology will be described.

(Entire structure of biomolecule detection apparatus)

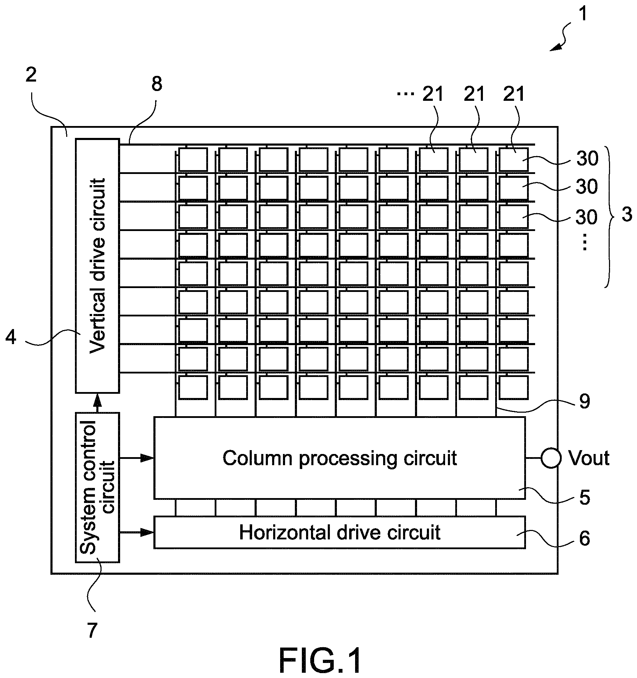

Fig. 1 is a schematic diagram showing the structure of a biomolecule detection apparatus 1 according to this embodiment. As shown in the figure, the biomolecule detection apparatus 1 is constituted of a chemical sensor 3 formed of a plurality of cells 30 arranged on a substrate 2 and a peripheral circuit for driving the chemical sensor 3. For each of the cells 30, a photodiode 21 is provided, as will be described later in detail.

The number of cells 30 and the arrangement thereof are not limited and can be changed as appropriate. Here, the cells 30 are arranged in a matrix pattern on a plane of the substrate 2. A row direction is set as a vertical direction, and a column direction is set as a horizontal direction.

The peripheral circuit is constituted of a vertical drive circuit 4, a column signal processing circuit 5, a horizontal drive circuit 6, and a system control circuit 7. Further, the photodiodes 21 of the cells 30 are connected to pixel drive lines 8 for each row and connected to vertical signal lines 9 for each column. The pixel drive lines 8 are connected to the vertical drive circuit 4, and the vertical signal lines 9 are connected to the column signal processing circuit 5.

The column signal processing circuit 5 is connected to the horizontal drive circuit 6, and the system control circuit 7 is connected to the vertical drive circuit 4, the column signal processing circuit 5, and the horizontal drive circuit 6. It should be noted that the peripheral circuit can be disposed on a position of being laminated on a pixel area or a position opposite to the substrate 2, for example.

The vertical drive circuit 4 is formed of a shift register, for example. The vertical drive circuit 4 selects the pixel drive line 8, supplies a pulse for driving the photodiodes 21 to the pixel drive line 8 selected, and drives the photodiodes 21 on a row basis. In other words, the vertical drive circuit 4 performs selective scanning for the photodiodes 21 in the vertical direction sequentially on the row basis. Then, through the vertical signal line 9 vertically wired with respect to the pixel drive line 8, the vertical drive circuit 4 supplies, to the column signal processing circuit 5, a pixel signal based on a signal charge generated in accordance with a received light quantity in the photodiodes 21.

The column signal processing circuit 5 performs a signal processing such as a noise removal for each pixel column with respect to a signal output from the photodiodes 21 of one row. In other words, the column signal processing circuit 5 performs signal processing such as a correlated double sampling (CDS), a signal amplification, and an analog/digital (AD) conversion for removing a fixed pattern noise specific to a pixel.

The horizontal drive circuit 6 is formed of the shift register, for example, and sequentially outputs horizontal scanning pulses, thereby selecting the column signal processing circuits 5 in order and causing each column signal processing circuit 5 to output a pixel signal.

The system control circuit 7 receives an input clock and data that specifies an operation mode or the like and outputs data relating to inside information or the like of the chemical sensor 3. That is, on the basis of a vertical synchronizing signal, a horizontal synchronizing signal, and a master clock, the system control circuit 7 generates a clock signal and a control signal which are references of the operations of the vertical drive circuit 4, the column signal processing circuit 5, the horizontal drive circuit 6, and the like. Then the system control circuit 7 inputs those signals to the vertical drive circuit 4, the column signal processing circuit 5, the horizontal drive circuit 6, and the like.

As described above, the vertical drive circuit 4, the column signal processing circuit 5, the horizontal drive circuit 6, the system control circuit 7, and a pixel circuit (to be described later) provided to the photodiodes 21 constitute a drive circuit that drives the photodiodes 21.

(Structure of chemical sensor)

The structure of the chemical sensor 3 will be described.

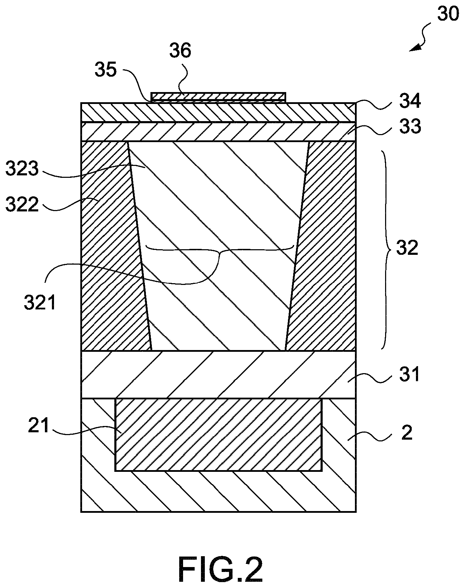

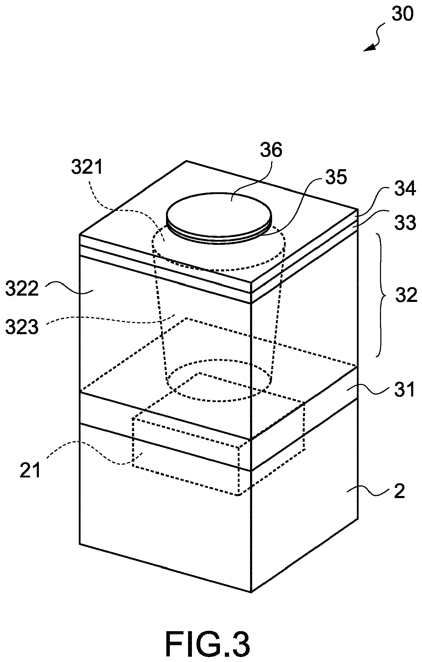

Figs. 2 and 3 are schematic diagrams each showing one cell 30 of the chemical sensor 3 according to this embodiment. Fig. 2 is a cross-sectional view of the cell 30, and Fig. 3 is a perspective view of the cell 30. As shown in the figures, the chemical sensor 3 includes a protection insulating layer 31, an optical layer 32, a flattening layer 33, an intermediate layer 34, an adhesive layer 35, and a probe material layer 36 laminated on the substrate 2.

The substrate 2 is made of single crystal silicon, for example, and has a light receiving surface on one principal surface side of the substrate 2. On a surface layer on the light receiving surface side, the photodiodes 21 each formed of an impurity region are formed and arranged two-dimensionally. The photodiode 21 is provided for each of the cells 30.

It should be noted that the photodiodes 21 may be provided only on the one principal surface side as the light receiving surface side of the substrate 2 as shown in the figure or may be provided over the one principal surface side and another principal surface side. The chemical sensor 3 can have an element structure of a CMOS (Complementary Metal Oxide Semiconductor) or a CCD (Charge Coupled Device) type. When necessary, another impurity region such as a floating diffusion or an element isolation (not shown) is disposed therein.

Further, on the substrate 2 provided with the impurity region including the photodiode 21, a gate insulating film (not shown), a gate electrode (not shown), or the like may be disposed. In this case, the protection insulating layer 31 is disposed so as to cover the gate insulating film or the gate electrode. Further, a pixel circuit including the gate insulating film or the gate electrode may be disposed on a side opposite to the light receiving surface of the substrate 2.

The optical layer 32 is a layer for guiding fluorescence to be described later to the photodiode 21. As shown in Figs. 2 and 3, in the optical layer 32, a waveguide 321 is formed. The waveguide 321 is structured to guide the fluorescence to the photodiode 21 and is formed toward the photodiode 21. In this embodiment, the waveguide 321 is formed by forming a through hole in a metal layer 322 made of metal (such as Al and Cu) having light reflectiveness, and is therefore surrounded by a reflection surface having the light reflectiveness.

Further, the waveguide 321 can have such a tapered shape that a diameter thereof is gradually reduced to the photodiode 21 side. With this structure, it is possible to condense light to the photodiode 21 while guiding the fluorescence that is isotropically emitted to the waveguide in a wide range.

In the waveguide 321, a spectral filter 323 made of a spectral material is provided. The spectral material is a material for attenuating excitation light and causing fluorescence to pass therethrough and can be selected as appropriate in accordance with wavelengths of the excitation light and the fluorescence. As the spectral filter 323, a color filter for causing fluorescence of a predetermined wavelength band to pass therethrough can be used. For the color filter, a color filter using a pigment or a dye is desirable.

The metal layer 322 and the spectral filter 323 form a clad layer that reflects light and a core layer in which light is transmitted. At a time of transmission in the spectral filter 323 as the core layer, the excitation light is transmitted while being absorbed and attenuated, and the fluorescence is transmitted to the PD while being reflected on the clad layer with a low loss. Because the fluorescence from a biomaterial is isotropic, a waveguide structure that uses a refractive index difference, such as an optical fiber, cannot transmit fluorescence having an angle component equal to or more than a critical angle, with the result that a fatal problem for the chemical sensor occurs in that a sufficient detection signal intensity is not obtained, and a crosstalk into an adjacent pixel occurs to detect a noise.

In view of this, in the present technology, for the clad layer, the metal material such as Al and Cu is used, thereby increasing an angle component which can be taken and significantly reducing the crosstalk to the adjacent pixel. It should be noted that, for the clad layer, it is necessary to select a material which has sufficiently high reflectance with respect to a fluorescent light wavelength.

The flattening layer 33 is a layer for flattening the optical layer 32. On an upper surface of the optical layer 32, unevenness may be generated when the spectral filter 51 is formed. The flattening layer 33 is a layer for flattening the unevenness in order to form the upper layers (intermediate layer 34 and the like). For a material of the flattening layer 33, an organic-based material having a high adhesiveness with respect to the metal material and the spectral material that form the optical layer 32 is desirable.

The intermediate layer 34 is a layer for regionally selectively forming the adhesive layer 35 and the probe material layer 36 formed thereon. Specifically, by a process to be described later, a surface treatment can be performed in such a manner that the adhesive layer 35 is formed only on a predetermined area of the intermediate layer 34. The intermediate layer 34 can be formed so that an area where the adhesive layer 35 is intended to be formed (hereinafter, referred to as probe holding area) is set to be hydrophilic, and the other area is formed of a silicon oxide or a silicon nitride to be hydrophobic, for example.

The probe holding area is desirably formed immediately above the photodiode 21 so as to have a size smaller than a diameter of the waveguide 321, that is, such a size that one probe holding area is entirely opposed to the waveguide 321. With this structure, the fluorescence generated from the probe material layer 36 can be efficiently caused to enter the waveguide 321.

The adhesive layer 35 is a layer for holding the probe material layer 36. As described above, the adhesive layer 35 is laminated only on the probe holding area of the intermediate layer 34 and holds the probe material layer 36 only above the probe holding area. The adhesive layer 35 can be formed of a biomolecule adhesive capable of being selectively adsorbed only to the probe holding area and causing a probe material (biomolecule) to adhere thereto. As an example of such a material, fibronectin can be given.

The probe material layer 36 is made of a probe material appropriately selected from biomolecules (DNA, RNA, a protein, various antigens, or the like) in accordance with the target material to be detected. The probe material layer 36 is formed by causing the probe material to adhere to the adhesive layer 35, that is, formed only above the probe holding area where the adhesive layer 35 is formed.

The chemical sensor 3 is formed by the cells 30 each having the structure as described above. It should be noted that the chemical sensor 3 may be supplied to a user without being provided with the probe material layer 36. In this case, the user can use any probe material with the material adhered to the adhesive layers 35.

Further, the chemical sensor 3 may be supplied to the user without being provided with the adhesive layer 35 and the probe material layer 36. In this case, the user can use any biomolecule adhesive and probe material to use the chemical sensor 3. In this case, the surface treatment as described above is also performed for the intermediate layer 34, so it is possible for the user to cause the adhesive to be adsorbed only to the probe holding area, that is, cause the probe material to adhere only to the probe holding area.

(Method of producing chemical sensor)

A method of producing the chemical sensor 3 having the structure as described above will be described.

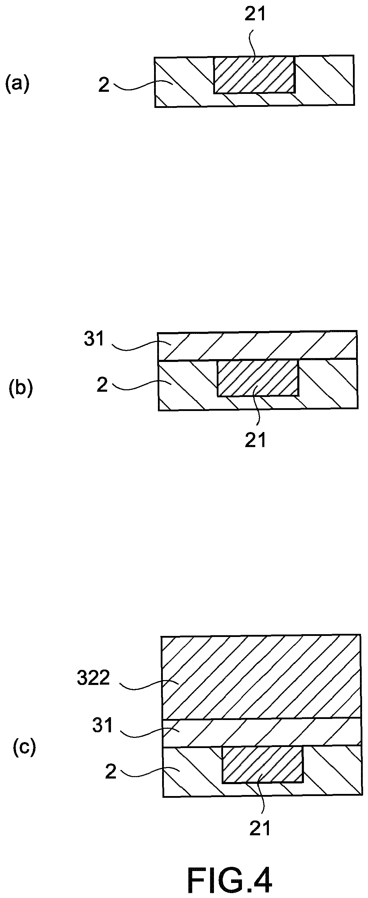

Figs. 4 to 7 are schematic diagrams showing the method of producing the chemical sensor 3. It should be noted that in these figures, one cell 30 is shown, but in actuality, it is possible to produce the plurality of cells 30 arranged on the substrate 2 at the same time.

First, as shown in Fig. 4(a), in the substrate 2 formed of single crystalline silicon or the like, the photodiode 21 is formed. The photodiode 21 can be formed by forming an impurity region by an ion implantation and a thermal treatment and forming a gate insulating film, a gate electrode, and the like (not shown) in the substrate 2. As described above, the photodiodes 21 can be arranged on the substrate 2 in a matrix pattern.

Subsequently, as shown in Fig. 4(b), on the substrate 2 in which the photodiode 21 is formed, the protection insulating layer 31 is formed by any film formation method. Further, as shown in Fig. 4(c), the metal layer 322 is formed on the protection insulating layer 31. The metal layer 322 can be formed by a sputtering method, for example.

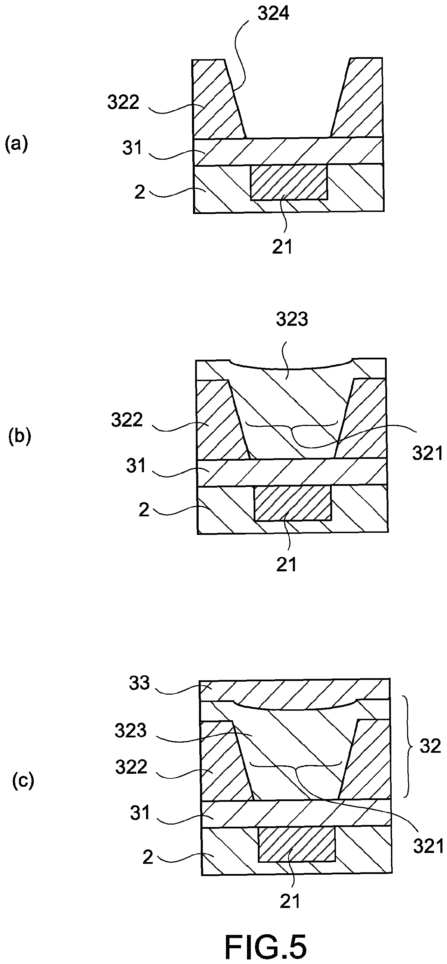

Subsequently, as shown in Fig. 5(a), the metal layer 322 above each of the photodiodes 21 is patterned, thereby forming an opening 324 serving as the waveguide 321. The patterning of the metal layer 322 can be performed by lithography or dry etching, for example. Then, as shown in Fig. 5(b), in the opening 324, the spectral material is filled, thereby forming the spectral filter 323. At this time, on an upper surface of the spectral filter 323, unevenness may be generated. By forming the spectral filter 323 in the opening 324, the waveguide 321 is formed.

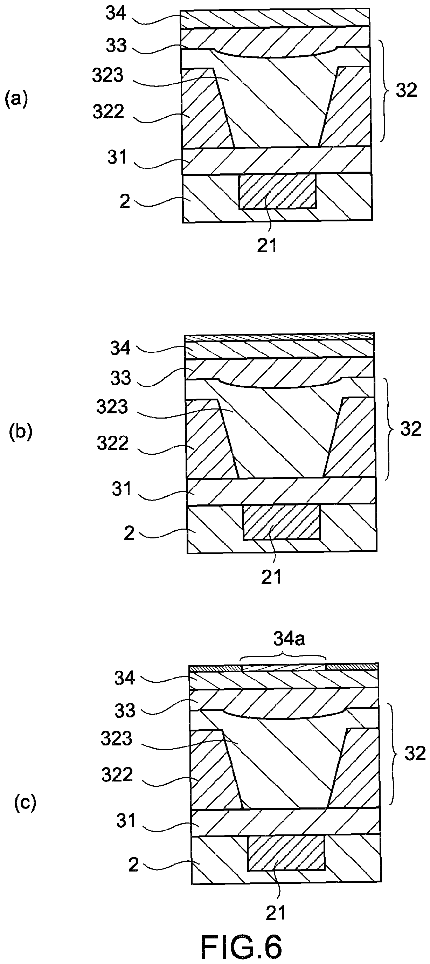

Then, as shown in Fig. 5(c), the spectral filter 323 is coated with a flattening material, and a baking treatment is performed when necessary, thereby forming the flattening layer 33. Further, on the flattening layer 33, the intermediate layer 34 is formed. The intermediate layer 34 can be formed by laminating a material such as a silicon oxide on the flattening layer 33 as shown in Fig. 6(a) and laminating a photosensitive silane coupling agent as shown in Fig. 6(b).

Further, as shown in Fig. 6(c), the intermediate layer 34 is partially irradiated with ultraviolet light through a photo mask, thereby forming a probe holding area 34a. The photosensitive silane coupling agent is irradiated with the ultraviolet light, with the result that the property thereof is changed to be hydrophilic. Thus, by selectively irradiating an area where the probe holding area 34a is intended to be provided by photolithography with the ultraviolet light, it is possible to set the area to be hydrophilic and the other area to be hydrophobic.

In this embodiment, the probe holding area 34a can be formed immediately above each of the photodiodes 21 so as to have a size smaller than the diameter of the waveguide 321, that is, have such a size that one probe holding area is entirely opposed to the waveguide 321.

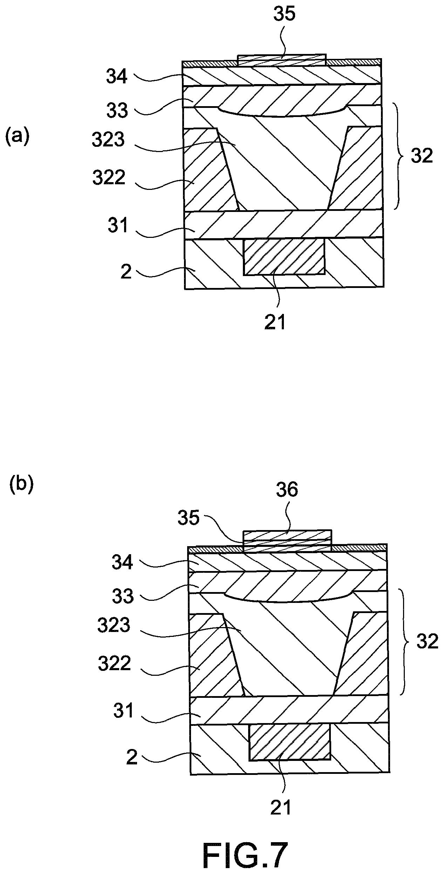

Further, to laminate the adhesive layer 35 and the probe material layer 36, the following process can be performed. As shown in Fig. 7(a), on the probe holding area 34a of the intermediate layer 34, the adhesive layer 35 is formed. Because the probe holding area 34a is hydrophilic as described above, the hydrophilic biomolecule adhesive (fibronectin or the like) is brought into contact therewith, with the result that the biomolecule adhesive remains only on the probe holding area 34a.

Then, as shown in Fig. 7(b), when any probe material is brought into contact onto the adhesive layer 35, the probe material is adhered to the adhesive layer 35, thereby forming the probe material layer 36. On an area where the adhesive layer 35 is not provided, the probe material is not adhered, and therefore the probe material layer 36 is not formed, Thus, it is possible to arbitrarily set the area where the probe material layer 36 is intended to be formed by the surface treatment for the intermediate layer 34.

As described above, it is possible to produce the chemical sensor 3.

(Characteristic of chemical sensor)

A spectral characteristic of the chemical sensor 3 having the structure as described above will be described. For the chemical sensor 3 having parameters shown in Table 1 below, numerical calculations are performed, and a signal component (fluorescent intensity) and a noise component (excitation light) are evaluated with an electromagnetic field intensity of a surface layer of the photodiode as an indicator. It should be noted that the thicknesses of the probe material and the target material are not reflected on a calculation model because of their nano-order thicknesses.

[Table 1]

| constituent element | size | refractive index @ 550 nm |
| --- | --- | --- |
| cell size | 3.0 $\mu$m$\square$ | - |
| protection insulating layer | 500 nmt | 1.5 |
| metal layer | 1.5 $\mu$m$\varphi$ - 2.0 $\mu$m$\varphi$ | 0.96+6.69i |
| spectral filter | 1.5 $\mu$m$\varphi$ - 2.0 $\mu$m$\varphi$ | 1.65+0.3i |
| flattening layer | 200 nmt | 1.5 |
| intermediate layer | 200 nmt | 1.45 |
| angle of incident: 0°, 5°, 15°, 45° | | |

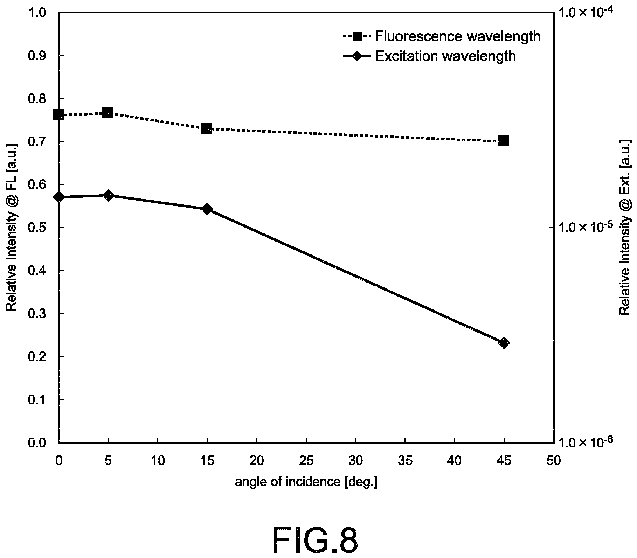

Simulation results are shown in Fig. 8. On a lateral axis, the angle of incidence is plotted. On longitudinal axes, the signal component (fluorescent intensity) and the noise component (excitation light) are plotted in arbitrary units. From the result of the fluorescent intensity (broken line, left Y axis), it can be understood that angle dependency is suppressed to be low, which is an effect of the waveguide 321. Further, for the excitation intensity (solid line, right Y axis), the larger the angle of incidence, the larger the attenuation becomes. This is because a light path length of light that is transmitted in the spectral filter 323 becomes longer. In addition, an SN (signal/noise) ratio is of the order of $10^4$, so it can be understood that highly accurate detection can be performed.

(Cell arrangement of chemical sensor)

As described above, the chemical sensor 3 is formed of the cells 30, but it is also possible to form the chemical sensor 3 by arranging cells for reference. Hereinafter, the assumption is made that the cell 30 having the structure as described above is set as a detection cell 30a, and the chemical sensor 3 is formed of reference cells 30b and black cells 30c in addition to the detection cells 30a.

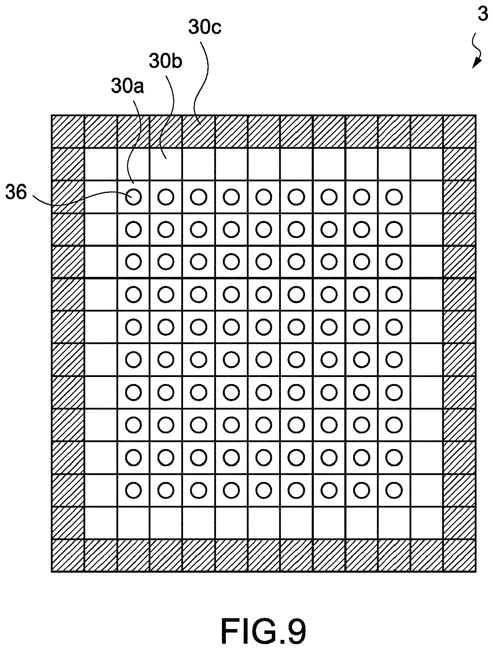

Fig. 9 is a diagram showing the chemical sensor 3 viewed from an upper surface side (probe material layer 36 side). As shown in the figure, the chemical sensor 3 is constituted of the detection cells 30a, the reference cells 30b, and the black cells 30c. The detection cells 30a each have the structure described above. Any number of detection cells 30a may be provided, but a mega-pixel size is desirable.

The reference cell 30b has the structure in which the adhesive layer 35 and the probe material layer 36 are not formed in the structure of the detection cell 30a. The reference cells 30b can be disposed around the detection cells 30a, for example. As described above, the probe holding area where the adhesive layer 35 is formed can be arbitrarily set by the surface treatment for the intermediate layer 34, so in order to form the reference cell 30b, a pixel without the probe holding area may be formed.

Although details will be described later, the detection cells 30a are irradiated with the excitation light, and on the probe material layer 36, fluorescence caused by binding of the probe material and the target material is generated. The excitation light is blocked by the spectral filter 323 of the waveguide 321, and only the fluorescence reaches the photodiode 21 and is detected. Here, the excitation light is not completely blocked by the spectral filter 323 and thus may be detected by the photodiode 21. Therefore, the reference cell 30b in which the probe material layer 36 is not formed detects the amount of leakage of the excitation light, and the detection can be used to correct a detection result of the detection cell 30a.

The black cell 30c has the structure in which the opening 324 is not formed in the metal layer 322 in the structure of the cell 30 described above, that is, the waveguide 321 is completely blocked by the metal layer 322. The black cells 30c can be disposed around the reference cells 30b, for example. The black cell 30c is used for definition of a black level of the photodiode which can be affected by a temperature or the like.

The chemical sensor 3 can have the pixel arrangement as described above. The arrangements of the detection cells 30a, the reference cells 30b, and the black cells 30c and the numbers of those are not limited to the example described above and can be changed as necessary.

(Biomolecule detection method using chemical sensor)

A biomolecule detection method by using the chemical sensor 3 described above will be described. The assumption is made that the chemical sensor 3 includes the probe material layer 36 made of any probe material in each of the detection cells 30a.

In the detection of the target material, for example, in the case where DNA is used as the probe material, a 5'-fluorescein-labeled DNA is used as an example. If a measurement material contains DNA having a sequence complementary to the 5'-fluorescein-labeled DNA, a hybridization reaction occurs, resulting in a change of the probe material from single-stranded DNA (ss-DNA) to double-stranded DNA (ds-DNA). The change causes a permittivity of the surrounding of a fluorescent molecule to change. Thus, it is possible to detect the change in emission wavelength and intensity of the fluorescence by the photodiode 21.

Further, in the case where the DNA is used as the probe material, DNA which is not fluorescently labeled is used as the probe material, and the 5'-fluorescein-labeled DNA is used for a sample, for example. In this case, if the sample contains DNA having a sequence complementary to the DNA as the probe material, the hybridization reaction occurs, resulting in a change into ds-DNA fluorescently labeled. The emission of the fluorescence from the fluorescence label can be detected by the photodiode 21.

Alternatively, in the case where the DNA is used as the probe material, DNA which is not fluorescently labeled is used for the probe material, and a fluorescence pigment is not also introduced into the sample side. In this case, if the sample contains DNA having a sequence complementary to the DNA as the probe material, the hybridization reaction occurs, resulting in a change into ds-DNA. Then, a treatment of fluorescent labeling by selectively dying only the ds-DNA is performed by using Pico-Green 2-stranded DNA quantification reagent produced by Molecular Probes, thereby introducing the fluorescent label into the ds-DNA part. The emission of the fluorescence from the fluorescent label is detected by the photodiode 21.

As described above, in the state in which the probe material, the target material, or a bound body of the probe material and the target material is fluorescently labeled, when the chemical sensor 3 is irradiated with the excitation light, the fluorescence is generated from the fluorescent label, or the wavelength and the intensity of the fluorescence are changed. For the irradiation with the excitation light, the following structure is desirable.

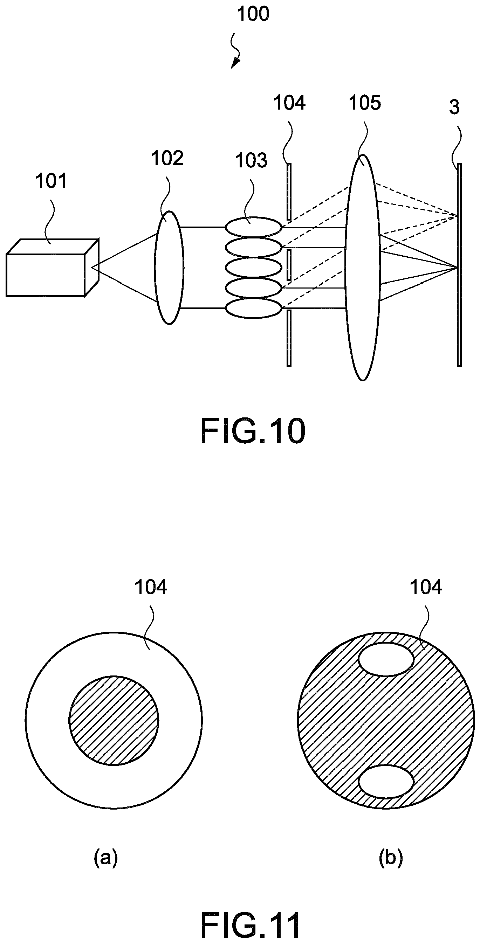

Fig. 10 is a schematic diagram showing the structure of an excitation light irradiation apparatus 100. As shown in the figure, the excitation light irradiation apparatus 100 includes an excitation light source 101, a collimator lens 102, a fly eye lens 103, a diaphragm 104, and a condenser lens 105. The arrangement thereof is well known as a Kohler illumination.

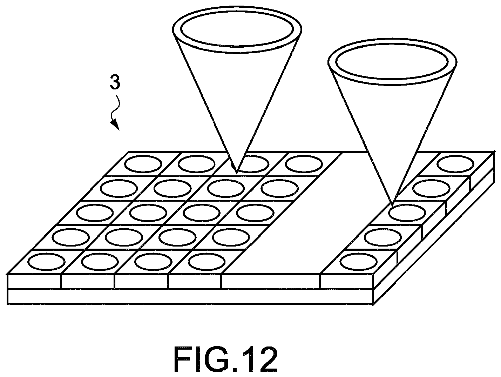

Figs. 11 are schematic diagrams each showing an example of the shape of the diaphragm 104. Shaded areas are light-shielding portions. In the following description, the shape of the diaphragm shown in Fig. 11(a) is used, but the shape of the diaphragm as shown in Fig. 11(b) can also be used. Fig. 12 is a schematic diagram showing the shape of illumination light.

Excitation light (laser or halogen light) emitted from the excitation light source 101 is converted into parallel light and made to be uniform by the fly eye lens (compact lens array) 103. Light that exits the fly eye lens 103 is converted into an annular secondary light source shape by the diaphragm 104 shown in Fig. 11(a) and condensed on the chemical sensor 3 by the condenser lens 105. With this structure, the excitation light is emitted with uniform intensity, illumination shape, and illumination angle irrespective of the position on the chemical sensor 3.

Here, the shape of the excitation light is converted into the annular shape by the diaphragm 104, with the result that the excitation light enters the cells 30 of the chemical sensor 3 in an oblique direction. As described in the interpretation of the simulation result, because of the oblique-incidence illumination, a distance by which the excitation light passes in the spectral filter 323 becomes longer as compared to the case where the excitation light enters the cells 30 in a vertical direction, and is therefore sufficiently attenuated by the spectral filter 323. Thus, by using the excitation light that is incident obliquely (oblique-incidence light), it is possible to attenuate the excitation light intensity and maintain the fluorescence intensity, so a high SN ratio can be obtained.

That is, the method of illuminating the excitation light shown in Fig. 10 is particularly effective to the case of using the chemical sensor 3 according to this embodiment. It should be noted that the incident light to the chemical sensor 3 is not limited to have the annular shape, and any oblique-incidence shape can be used, which can be adjusted by the shape of the diaphragm 104.

(Signal processing by biomolecule detection apparatus)

As described above, the fluorescence generated by irradiating the chemical sensor 3 with the excitation light is detected by the photodiodes 21, thereby making it possible to detect the target material. A signal processing operation by the biomolecule detection apparatus 1 at this time will be described with the chemical sensor 3 shown in Figs. 4 as an example.

An output signal of the photodiode 21 of a specific cell 30 is represented by a signal Is, an output signal of the photodiode 21 of a specific reference cell 30b is represented by a signal Iref, and an output signal of the photodiode 21 of a specific black cell 30c is represented by a signal Ib. First, as shown in the following (Expression 1), a black level is subtracted in the same way as image processing in a normal image sensor, and a transform is performed as shown in (Expression 2), to perform standardization to a normal AD conversion scale.

$$\text{Is} = \text{Is-Ib} \qquad \text{(Expression 1)}$$

$$\text{Iref} = \text{Iref-Ib} \qquad \text{(Expression 2)}$$

Then, as shown in the following (Expression 3), a difference between the signal Is and the signal Iref is calculated, thereby canceling a leakage component of the excitation light to extract only a fluorescent component Ifl.

$$\text{Ifl} = \text{Is-Iref} \qquad \text{(Expression 3)}$$

In this way, it is possible to obtain the light component Ifl for each cell 30 with high accuracy.

As described above, according to this embodiment, the fluorescence caused by the binding of the target material specifically bound to the probe material is guided by the waveguides 321 to the photodiodes 321, so it is possible to detect the fluorescence with high accuracy. Further, by surrounding the waveguide 321 by the reflection surface having the light reflectivity, the fluorescence that enters the waveguides 321 is reflected by the reflection surfaces, so it is possible to detect the fluorescence irrespective of the angle of incidence and prevent the fluorescence from reaching the adjacent cell 30 by the reflection surface.

Further, the waveguide 321 has the tapered shape in such a manner that the diameter thereof is gradually reduced from the intermediate layer side 34 to the photodiode 21 side, with the result that it is possible to condense the fluorescence to the photodiode 21 while guiding the fluorescence that is isotropically emitted to the waveguide 321 in the wide range. Further, by forming the spectral filter 323 made of the spectral material that attenuates the excitation light and causes the fluorescence to pass therethrough in the waveguide 321, it is possible to prevent the excitation light from reaching the photodiode 21.

Further, by forming the probe holding area 34a so as to have the size opposed to the waveguide 321, the generation area of the fluorescence is smaller than the incidence opening of the waveguide 321, so it is possible to guide most of the fluorescence to the waveguide 321 and prevent the fluorescence from reaching the adjacent cell 30. In addition, by forming the probe holding area 34a only on a part of the cells 30, the cells 30 where the probe holding area 34a is not formed can be used for reference of the excitation light leaked and can be used to correct the fluorescence detection signal.

(Second embodiment)

A biomolecule detection apparatus according to a second embodiment of the present technology will be described. It should be noted that this embodiment is different from the first embodiment in terms of the pixel arrangement of the chemical sensor, but the other structure is the same as the first embodiment, and a description thereof will be omitted.

(Pixel arrangement of chemical sensor)

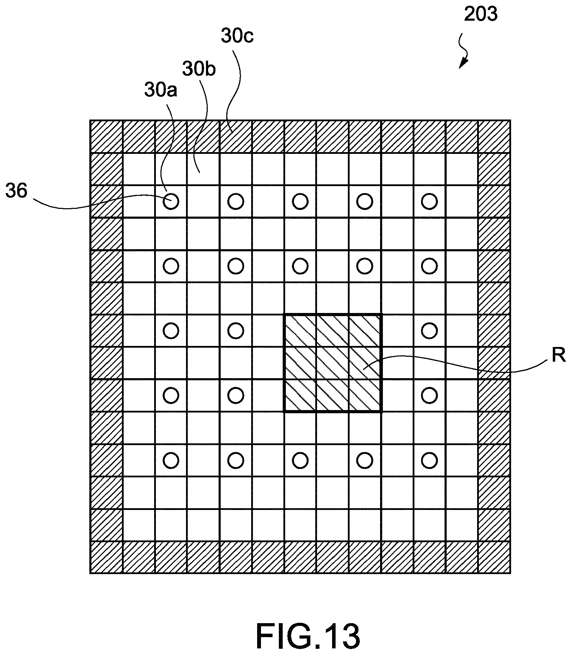

Fig. 13 is a schematic diagram showing a chemical sensor 203 according to this embodiment when viewed from above. As shown in the figure, the chemical sensor 203 can be constituted of the detection cells 30a, the reference cells 30b, and the black cells 30c. The detection cell 30a, the reference cell 30b, and the black cell 30c each have the same structure as the first embodiment, but in this embodiment, the reference cells 30b are disposed between the detection cells 30a so that the detection cells 30 are not next to each other.
With this structure, a distance between the adjacent detection cells 30a is 2 pixel pitch (XY direction) and $2\sqrt{2}$ pixel pitch (diagonal). Therefore, it is possible to significantly reduce a crosstalk to the adjacent detection cell 30a due to the fluorescence from the detection cell 30a, which increases the detection accuracy.
Further, as shown in a shaped part of Fig. 13, a reference area R constituted of the reference cells 30b sufficiently distanced from the detection cells 30a is provided, and only an output signal from the reference cells 30b can be set as the reference signal (Iref mentioned above). The position of the reference area R and the number thereof can be changed as necessary.
As described above, in this embodiment, by distancing the detection cells 30a from each other and disposing the reference cells 30b therebetween, it is possible to prevent the crosstalk between the adjacent detection cells 30a and detect the biomolecule with high accuracy.

(Third embodiment)

A biomolecule detection apparatus according to a third embodiment of the present disclosure will be described. It should be noted that this embodiment is different from the first embodiment in the element structure and the pixel arrangement of the chemical sensor, but the other structure of this embodiment is the same as the first embodiment, so a description thereof will be omitted.

(Pixel arrangement of chemical sensor)

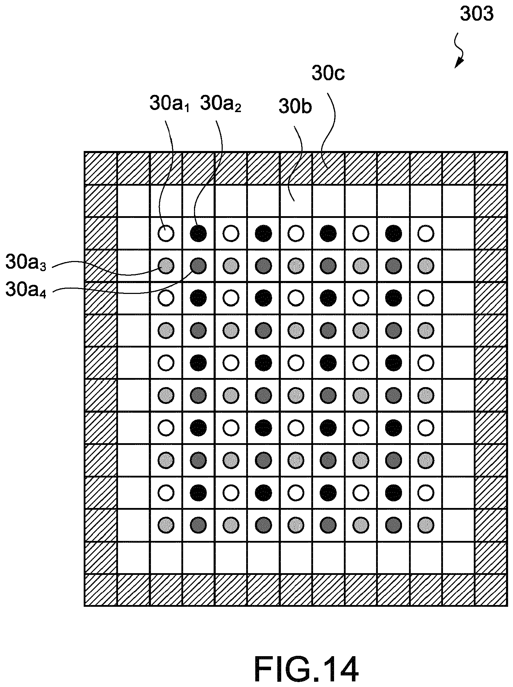

Fig. 14 is a schematic diagram showing a chemical sensor 303 according to this embodiment when viewed from above. As shown in the figure, the chemical sensor 303 can be constituted of the detection cells 30a, the reference cells 30b, and the black cells 30c.
In the detection cell 30a according to this embodiment, the spectral filter 323 is a color filter that causes only fluorescence of a predetermined band to pass therethrough, and color filters of four kinds having different cutoff wavelengths (shorter wavelength side relative to $\lambda n$ is blocked, and longer wavelength side relative to $\lambda n$ is transmitted) are used therefor. That is, for the chemical sensor 303, a first detection cell $30a_1$ having a color filter of a first color ($\lambda 1$), a second detection cell $30a_2$ having a color filter of a second color ($\lambda 2$), a third detection cell $30a_3$ having a color filter of a third color ($\lambda 3$), and a fourth detection cell $30a_4$ having a color filter of a fourth color ($\lambda 4$) are used. It should be noted that the kinds (colors) of the color filters are not limited to four but may be three or less or five or more.
As shown in Fig. 14, the first detection cell $30a_1$ is disposed so as to be next to only the second detection cell $30a_2$, the third detection cell $30a_3$, and the fourth detection cell $30a_4$ and not to be next to the first detection cell $30a_1$. The same holds true for the second detection cell $30a_2$, the third detection cell $30a_3$, and the fourth detection cell $30a_4$.
With this structure, it is possible to prevent the crosstalk between the detection cells $30a_n$ (n = 1, 2, 3, and 4) of the same kind. Specifically, in the illumination apparatus as shown in Fig. 10, a wavelength variable laser or a band pass filter may be operated in chronological order to emit light of $\lambda 1$, $\lambda 2$, $\lambda 3$, and $\lambda 4$ in this order or may be emitted at the same time by a wavelength light source. For the detection system as a whole, cost effectiveness can of course be achieved, and the detection can be possible with a plurality of wavelengths. Therefore, it is possible to perform a more multidirectional analysis and perform an analysis with a common sample, with the result that such an effect that a highly reliable analysis can be performed.
The present disclosure is not limited to the above embodiments and can be modified without departing from the scope of the invention as defined by the attached claims.
Description of Reference Numerals

1 biomolecule detection apparatus
2 substrate
3 chemical sensor
21 photodiode
31 protection insulating layer
32 optical layer
33 flattening layer
34 intermediate layer
35 adhesive layer
36 probe material layer


**Claims**

1. A chemical sensor (3), comprising:

    a substrate (2) on which a plurality of photodiodes (21) are arranged in a planar form;
    an optical layer (32) laminated on the substrate, the optical layer having a waveguide (321)formed therein for each photodiode, the waveguide for guiding fluorescence to a corresponding photodiode; and
    an intermediate layer (34) laminated on the optical layer, the intermediate layer having a probe holding area (35) formed thereon for each waveguide, the probe holding area being capable of holding a probe material, **characterized in that** the waveguide has a spectral filter (323) formed therein which attenuates excitation light and is made of a spectral material that causes the fluorescence to pass therethrough, and
    wherein the spectral filter is a color filter that causes a part of a wavelength of the fluorescence to pass therethrough, and
    wherein the color filter has a transmission wavelength different from that formed in a waveguide adjacent thereto.

2. The chemical sensor (3) according to claim 1, wherein
the waveguide (321) is surrounded by a reflection surface having light reflectiveness.

3. The chemical sensor (3) according to claim 2, wherein
the waveguide (321) has a tapered shape in such a manner that a diameter thereof is gradually reduced from the intermediate layer (34) side to the photodiode side (21).

4. The chemical sensor (3) according to claim 2, wherein
the probe holding area (35) is formed to have such a size as to be opposed to the waveguide (321).

5. The chemical sensor (3) according to claim 4, wherein
the probe holding area (35) is formed with respect to a part of the waveguide (321).

6. The chemical sensor (3) according to claim 2, further comprising
an adhesive layer (35) made of a biomolecule adhesive formed on the probe holding area (35).

7. The chemical sensor (3) according to claim 6, further comprising
a probe material layer (36) made of a probe material adhered onto the adhesive layer (35).

8. The chemical sensor (3) according to claim 7, wherein
the probe material is one of DNA, RNA, a protein, and an antigen.

9. A biomolecule detection apparatus (1), comprising:
the chemical sensor of claim 1; and a signal processing circuit (5) to process an output signal of each of the photodiodes of the chemical sensor.

10. The biomolecule detection apparatus (1) according to claim 9, wherein
the signal processing circuit (5) extracts a difference between the output signals of the photodiode (21) for which the probe holding area (35) is provided and the photodiode for which the probe holding area is not provided, as a signal corresponding to the fluorescence.

**11.** The biomolecule detection apparatus (1) according to claim 9, wherein the signal processing circuit (5) sets the output signal of the photodiode (21) for which light is shielded as a reference signal.

**12.** A biomolecule detection method, comprising:
preparing a chemical sensor (1) including a substrate (2) on which a plurality of photodiodes (21) are arranged in a planar form, an optical layer (32) laminated on the substrate, and an intermediate layer laminated on the optical layer, the optical layer having a waveguide (321) formed therein for each photodiode, the waveguide for guiding fluorescence to a corresponding photodiode, the intermediate layer having a probe holding area (35)formed thereon for each waveguide, the probe holding area being capable of holding a probe material
wherein the waveguide has a spectral filter (323) formed therein which attenuates excitation light and is made of a spectral material that causes the fluorescence to pass therethrough, and
wherein the spectral filter is a color filter that causes a part of a wavelength of the fluorescence to pass therethrough, and
wherein the color filter has a transmission wavelength different from that formed in a waveguide adjacent thereto;
forming an adhesive layer (35) made of a biomolecule adhesive on the probe holding area;
causing different probe materials to adhere to the adhesive layer to form a probe material layer;
bringing a measurement target substance into contact with the probe material layer to cause a target material contained in the measurement target substance to bind to the probe material;
removing the measurement target substance that is not bound to the target material; and
detecting fluorescence caused by binding of the target material to the probe material by the photodiodes.

**Patentansprüche**

**1.** Chemischer Sensor (3), umfassend:

ein Substrat (2), auf welchem eine Mehrzahl von Fotodioden (21) in planarer Form angeordnet ist;
eine optische Schicht (32), die auf das Substrat laminiert ist, wobei die optische Schicht einen darauf ausgebildeten Wellenleiter (321) für jede Fotodiode aufweist, wobei der Wellenleiter zum Leiten von Fluoreszenz zu einer entsprechenden Fotodiode ist; und eine Zwischenschicht (34), die auf die optische Schicht laminiert ist, wobei die Zwischenschicht eine darauf ausgebildete Sondenaufnahmefläche (35) für jeden Wellenleiter aufweist, wobei die Sondenaufnahmefläche zum Aufnehmen eines Sondenmaterials imstande ist, **dadurch gekennzeichnet, dass**
der Wellenleiter ein darin ausgebildetes Spektralfilter (323) aufweist, das Erregungslicht dämpft und aus einem Spektralmaterial hergestellt ist, das bewirkt, dass die Fluoreszenz dadurch durchtritt, und
wobei das Spektralfilter ein Farbfilter ist, das bewirkt, dass ein Teil einer Wellenlänge der Fluoreszenz dadurch durchtritt, und
wobei das Farbfilter eine Durchlasswellenlänge aufweist, die von der, die in einem dazu benachbarten Wellenleiter gebildet wird, verschieden ist.

**2.** Chemischer Sensor (3) nach Anspruch 1, wobei der Wellenleiter (321) von einer Reflexionsfläche mit Lichtreflexionsvermögen umgeben ist.

**3.** Chemischer Sensor (3) nach Anspruch 2, wobei der Wellenleiter (321) eine konisch zulaufende Form aufweist, derart dass ein Durchmesser davon von der Seite der Zwischenschicht (34) zur Fotodiodenseite (21) schrittweise kleiner wird.

**4.** Chemischer Sensor (3) nach Anspruch 2, wobei die Sondenaufnahmefläche (35) so ausgebildet ist, dass sie solch eine Größe aufweist, dass sie dem Wellenleiter (321) gegenüberliegt.

**5.** Chemischer Sensor (3) nach Anspruch 4, wobei die Sondenaufnahmefläche (35) in Bezug auf einen Teil des Wellenleiters (321) ausgebildet ist.

**6.** Chemischer Sensor (3) nach Anspruch 2, ferner umfassend:
eine Haftschicht (35), die aus einem Biomolekül-Haftmittel hergestellt und auf der Sondenaufnahmefläche (35) ausgebildet ist.

**7.** Chemischer Sensor (3) nach Anspruch 6, ferner umfassend:
eine Sondenmaterialschicht (36), die aus einem Sondenmaterial hergestellt und an die Haftschicht (35) geklebt ist.

**8.** Chemischer Sensor (3) nach Anspruch 7, wobei das Sondenmaterial eines von DNA, RNA, einem Protein und einem Antigen ist.

**9.** Vorrichtung zur Erkennung von Biomolekülen (1), umfassend:
den chemischen Sensor nach Anspruch 1; und
eine Signalverarbeitungsschaltung (5) zum Verarbeiten eines Ausgangssignals einer jeden der Fotodioden des chemischen Sensors.

**10.** Vorrichtung zur Erkennung von Biomolekülen (1) nach Anspruch 9, wobei
die Signalverarbeitungsschaltung (5) eine Differenz zwischen den Ausgangssignalen der Fotodiode (21), für welche die Sondenaufnahmefläche (35) vorgesehen ist, und der Fotodiode, für welche die Sondenaufnahmefläche nicht vorgesehen ist, als ein Signal extrahiert, das der Fluoreszenz entspricht.

**11.** Vorrichtung zur Erkennung von Biomolekülen (1) nach Anspruch 9, wobei
die Signalverarbeitungsvorrichtung (5) das Ausgangssignal der Fotodiode (21), für welche Licht abgeschirmt wird, als ein Referenzsignal festlegt.

**12.** Verfahren zur Erkennung von Biomolekülen, umfassend:

Herstellen eines chemischen Sensors (1), der ein Substrat (2), auf welchem eine Mehrzahl von Fotodioden (21) in planarer Form angeordnet ist, eine optische Schicht (32), die auf das Substrat laminiert ist, und eine Zwischenschicht umfasst, die auf die optische Schicht laminiert ist, wobei die optische Schicht einen darin ausgebildeten Wellenleiter (321) für jede Fotodiode aufweist, der Wellenleiter zum Leiten von Fluoreszenz zu einer entsprechenden Fotodiode ist, und die Zwischenschicht eine darauf ausgebildete Sondenaufnahmefläche (35) für jeden Wellenleiter aufweist, wobei die Sondenaufnahmefläche zum Aufnehmen eines Sondenmaterials imstande ist,
wobei der Wellenleiter ein darin ausgebildetes Spektralfilter (323) aufweist, das Erregungslicht dämpft und aus einem Spektralmaterial hergestellt ist, das bewirkt, dass die Fluoreszenz dadurch durchtritt, und wobei das Spektralfilter ein Farbfilter ist, das bewirkt, dass ein Teil einer Wellenlänge der Fluoreszenz dadurch durchtritt, und
wobei das Farbfilter eine Durchlasswellenlänge aufweist, die von der, die in einem dazu benachbarten Wellenleiter gebildet wird, verschieden ist;
Bilden einer Haftschicht (35), die aus einem Biomolekül-Haftmittel hergestellt ist, auf der Sondenaufnahmefläche; Veranlassen, dass verschiedene Sondenmaterialien an die Haftschicht geklebt werden, um eine Sondenmaterialschicht zu bilden;
Bringen einer Messzielsubstanz in Kontakt mit der Sondenmaterialschicht, um ein in der Messzielsubstanz enthaltenes Zielmaterial zum Binden an das Sondenmaterial zu veranlassen;
Entfernen der Messzielsubstanz, die nicht an das Zielmaterial gebunden ist; und
Erkennen von Fluoreszenz, die durch Bindung des Zielmaterials an das Sondenmaterial durch die Fotodioden verursacht wird.

**Revendications**

**1.** Capteur chimique (3), comprenant :

un substrat (2) sur lequel une pluralité de photodiodes (21) sont disposées dans une forme planaire ;
une couche optique (32) appliquée sur le substrat, la couche optique ayant un guide d'ondes (321) formé à l'intérieur pour chaque photodiode, le guide d'ondes servant à guider la fluorescence jusqu'à une photodiode correspondante ; et
une couche intermédiaire (34) appliquée sur la couche optique, la couche intermédiaire ayant une zone de retenue de sonde (35) formée par-dessus pour chaque guide d'ondes, la zone de retenue de sonde pouvant retenir un matériau sonde,
**caractérisé en ce que**
le guide d'ondes a un filtre spectral (323) formé à l'intérieur qui atténue la lumière d'excitation et est constitué d'un matériau spectral qui laisse passer la fluorescence, et

dans lequel le filtre spectral est un filtre coloré qui laisse passer une partie d'une longueur d'onde de la fluorescence, et

dans lequel le filtre coloré a une longueur d'onde de transmission différente de celle formée dans un guide d'ondes adjacent à celui-ci.

2. Capteur chimique (3) selon la revendication 1, dans lequel
le guide d'ondes (321) est entouré par une surface de réflexion ayant une réflectivité de la lumière.

3. Capteur chimique (3) selon la revendication 2, dans lequel
le guide d'ondes (321) a une forme conique telle qu'un diamètre de celui-ci est progressivement réduit depuis le côté de la couche intermédiaire (34) jusqu'au côté de la photodiode (21).

4. Capteur chimique (3) selon la revendication 2, dans lequel
la zone de retenue de sonde (35) est formée pour avoir une taille telle qu'elle est en face du guide d'ondes (321) .

5. Capteur chimique (3) selon la revendication 4, dans lequel
la zone de retenue de sonde (35) est formée par rapport à une partie du guide d'ondes (321).

6. Capteur chimique (3) selon la revendication 2, comprenant en outre
une couche adhésive (35) constituée d'un adhésif pour biomolécules formé sur la zone de retenue de sonde (35).

7. Capteur chimique (3) selon la revendication 6, comprenant en outre
une couche de matériau sonde (36) constituée d'un matériau sonde collé sur la couche adhésive (35).

8. Capteur chimique (3) selon la revendication 7, dans lequel
le matériau sonde est soit de l'ADN, soit de l'ARN, soit une protéine, soit un antigène.

9. Appareil de détection de biomolécules (1), comprenant :

le capteur chimique de la revendication 1 ; et
un circuit de traitement du signal (5) pour traiter un signal de sortie de chacune des photodiodes du capteur chimique.

10. Appareil de détection de biomolécules (1) selon la revendication 9, dans lequel
le circuit de traitement du signal (5) extrait une différence entre les signaux de sortie de la photodiode (21) pour laquelle la zone de retenue de sonde (35) est fournie et la photodiode pour laquelle la zone de retenue de sonde n'est pas fournie, en tant que signal correspondant à la fluorescence.

11. Appareil de détection de biomolécules (1) selon la revendication 9, dans lequel
le circuit de traitement du signal (5) définit le signal de sortie de la photodiode (21) pour laquelle la lumière est bloquée comme un signal de référence.

12. Procédé de détection de biomolécules, comprenant les étapes suivantes :

préparer un capteur chimique (1) comportant un substrat (2) sur lequel une pluralité de photodiodes (21) sont disposées dans une forme planaire, une couche optique (32) appliquée sur le substrat, et une couche intermédiaire appliquée sur la couche optique, la couche optique ayant un guide d'ondes (321) formé à l'intérieur pour chaque photodiode, le guide d'ondes servant à guider la fluorescence jusqu'à une photodiode correspondante, la couche intermédiaire ayant une zone de retenue de sonde (35) formée par-dessus pour chaque guide d'ondes, la zone de retenue de sonde pouvant retenir un matériau sonde, le guide d'ondes ayant un filtre spectral (323) formé à l'intérieur qui atténue la lumière d'excitation et est constitué d'un matériau spectral qui laisse passer la fluorescence, et le filtre spectral étant un filtre coloré qui laisse passer une partie d'une longueur d'onde de la fluorescence, et le filtre coloré ayant une longueur d'onde de transmission différente de celle formée dans un guide d'ondes adjacent à celui-ci ; former une couche adhésive (35) constituée d'un adhésif pour biomolécules sur la zone de retenue de sonde ; faire adhérer différents matériaux sondes à la couche adhésive pour former une couche de matériau sonde ;

mettre une substance cible de mesure en contact avec la couche de matériau sonde pour provoquer la liaison d'un matériau cible contenu dans la substance cible de mesure au matériau sonde ;

retirer la substance cible de mesure qui n'est pas liée au matériau cible ; et

détecter la fluorescence provoquée par la liaison du matériau cible au matériau sonde avec les photodiodes.

EP 2 772 751 B1

FIG.1

FIG.2

FIG.3

(a)    2

21

(b)

31

2

21

(c)

322

31

2

21

# FIG.4

FIG.5

**FIG.6**

(a)

(b)

# FIG.7

FIG.8

FIG.9

100

104  105  3

101  102  103

FIG.10

104  104

(a)  (b)

FIG.11

FIG.12

FIG.13

FIG.14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002202303 A
- JP 2006004991 A
- WO 2011103504 A
- US 20080081769 A1